# EUROPEAN PATENT APPLICATION

(11) **EP 4 685 162 A1**
(43) Date of publication of application: **28.01.2026**
(21) Application number: 24190529.8
(22) Date of filing: 24.07.2024
(51) Int. Cl.: C07K 16/36, A61P 7/02, A61K 39/395

(54) **HUMAN ANTI-FV ANTIBODY, THERAPEUTIC AND DIAGNOSTIC APPLICATIONS THEREOF**

(71) Applicant: CEINGE Biotecnologie Avanzate Franco Salvatore s.c. a r.l., 80145 Napoli (IT)
(72) Inventor: PASSARIELLO, Margherita, 80145 NAPOLI (IT); LEONARDI, Antonio, 80138 NAPOLI (IT); DE LORENZO, Claudia, 80122 NAPOLI (IT)
(74) Representative: Bianchetti & Minoja with Trevisan & Cuonzo IPS SRL

(57) **Abstract**

The present invention discloses an anti-factor V antibody useful as an anticoagulant agent and as a tool for diagnostic and research applications. The antibody or its fragments can be conveniently used as anticoagulant agents inhibiting Factor V and/or Factor Va, particularly to reduce clot formation and associated thrombotic events, or they can be used *in vitro* to measure the levels of Factor V in a blood sample.

Other aspects of the invention regard nucleic acids encoding the antibody or fragments thereof as well as expression vectors and host cells containing them.

## Description

The present invention provides an anti-factor V antibody useful as an anticoagulant agent and as a tool for diagnostic and research applications. Other aspects of the invention regard nucleic acids encoding the antibody or fragments thereof, expression vectors and host cells containing them, as well as therapeutic, diagnostic and research applications of said antibody or fragments thereof.

### Background of the invention

Cardiovascular diseases are the leading cause of mortality worldwide, representing around one third of the registered deaths every year. There's a wide spectrum of etiological factors, both genetic and environmental, at the base of these diseases, which can have different implications and molecular mechanisms. Remarkably, many of these alterations ultimately converge into a common downstream manifestation: thrombosis. (Senst B, et al. StatPearls 2024*.*)

Thrombosis is the occlusion of a blood vessel caused by the formation of clots. Several imbalances can lead to this outcome, and they have been classified into three different groups of alterations named "Vyrchow's triad": vascular trauma, vascular stasis, and hypercoagulability. Notably, even cancer can cause hypercoagulability states as well, through the overexpression of procoagulant proteins and also the interaction of the tumor itself with the blood vessels endothelium. (Ashorobi D, et al. StatPearls; 2024)

Given that blood hypercoagulability significantly contributes to thrombogenesis, modulating the coagulation cascade is crucial in the management of these patients. Coagulation is a complex series of enzymatic reactions that culminate in the formation of a blood clot. This intricate cascade involves the activation of clotting factors in a precisely regulated sequence, ensuring rapid response to vascular injury while preventing unwarranted clot formation.

For decades, heparins (initially unfractionated, and later on low molecular weight heparins) and vitamin K antagonists (warfarin, phenprocoumon, acenocoumarol) have been employed in the treatment and prevention of thromboembolism (Franchini M, Blood Transfus. 2016)*.*

However, despite the excellent clinical outcomes achieved with traditional anticoagulants, the use of low molecular weight heparins (LMWH) still carries a risk of heparin-induced thrombocytopenia, and their long-term use as parenteral administration is limited in the clinical settings. Similarly, although vitamin K antagonists (VKA) are well-established in treating a wide range of thromboembolic disorders, their use is hindered by several limitations, including delayed onset and offset of action, genetic variations in metabolism, and interactions with food and other drugs, thus requiring frequent monitoring and dose adjustments (Sam Schulman, Nick R. Bijsterveld, Anticoagulants and Their Reversal, Transfusion Medicine Reviews, 2007)*.*

In the past 15 years, some researchers have been focusing on the new direct oral anticoagulants (DOACs), which selectively target specific steps of the coagulation cascade, for example the direct inhibition of thrombin (dabigatran) and inhibition of Factor Xa (edoxaban, apixaban, and rivaroxaban). Since the first FDA-approval in 2010, DOACs have now replaced VKA for different purposes thanks to their versatility, favorable pharmacological profile, and lack of need for monitoring. (Nun K. Bentounes, et al. Development of new anticoagulant in 2023: Prime time for anti-factor XI and XIa inhibitors, JMV)*.* Nonetheless, despite having a better safety profile than VKA, the risk of bleeding continues to be a concern with case-fatality rate of major bleeding of 8% [Chai-Adisaksopha Cet al. Mortality outcomes in patients receiving direct oral anticoagulants.... J Thromb Haemost 2015]*,* making necessary the development of specific antidotes. In 2015 FDA and EMA approved an antibody fragment, called idarucizumab, inhibiting dabigatran and in 2018/2019 Andexanet alfa, the inhibitor apixaban or rivaroxaban, for factor Xa. This highlights the ongoing need for more targeted and safer anticoagulant therapies.

Aptamers were also considered as candidates for this aim, due to their ability to bind to their target protein, with high affinity and specificity and their non-immunogenicity. Several aptamers targeting blood coagulation factors have been evaluated in clinical trials and have shown promising preclinical results. However, none of these aptamers have been approved for clinical use due to challenges related to their short half-life and rapid renal clearance. (Liu, M.; Int. J. Mol. Sci. 2021 ; Derszniak, K et al. Front. Pharmacol. 2019 ; Mao, Y Et al. Nucleic Acids Res. 2020).

The generation of monoclonal antibodies targeting specific coagulation factors allows to minimize off-target effects and the interaction with other proteins in the coagulation cascade as well as they have a longer half-life in circulation.

Factor V (FV) is a single-chain plasma glycoprotein containing multiple domains (A1, A2, A3 and C1, C2) and the large activation peptide (B domain) between A2 and A3. It represents the precursor or pro-cofactor of the Factor Va (FVa), which is generated by proteolysis and removal of the inhibitory B domain during activation of FV mediated by thrombin. FVa is the active form which acts as a cofactor for FXa, leading to prothrombin activation essential for efficient blood clotting [Ref. B. Dahlbäck et al. Novel insights into the regulation of coagulation by factor V isoforms, tissue factor pathway inhibitorα, and protein S. Journal of Thrombosis and Haemostasis. 2017; 15, 7, 1241-1250; Schreuder M, Reitsma PH, Bos MHA. Blood coagulation factor Va's key interactive residues and regions for prothrombinase assembly and prothrombin binding. J Thromb Haemost. 2019 Aug; 17(8): 1229-1239].

Inhibiting factor V can significantly attenuate thrombin generation, thereby reducing clot formation and associated thrombotic events.

### Description of invention

A fully human monoclonal antibody specific for Factor V was isolated by using an innovative strategy of selection based on phage display, starting from a large human scFv-phage library. The selection occurred by panning a repertoire of phages expressing human antibody fragments (scFv) on the purified recombinant protein in native form (FV) or subsequently activated by proteolytic maturation (FVa).

Phages expressing scFv specific for molecular targets have been isolated through cycles of parallel selections for affinity to each antigen immobilized on a solid surface. After massive screening through ELISA assays in parallel on FV and FVa, the positive phage clones, obtained by selection, were analyzed for their binding capacity to Factor V by ELISA assays. The clone with the highest affinity of binding was employed for the isolation of the cDNA employed for the expression of the antibody fragment (scFv) in soluble form in the periplasm of bacteria and confirmed its binding specificity.

The scFv fragment was then successfully converted into IgG1 and was found capable of binding to Factor V with good affinity (Kd 5-10 nM) both by ELISA and BLI assays. Its cross-reactivity for some other coagulation factors was found null or very poor. Furthermore, when tested in blood clotting tests, it was found to prolong activated partial thromboplastin time (aPTT).

Accordingly, the invention provides an antibody specifically binding to Human Factor V and Va, or a fragment thereof selected from Fab, Fab', Fab'-SH, Fv, scFv, (Fab')2, diabodies (dAb) or single domain antibody (sdAb), wherein said antibody or fragment thereof comprises a heavy chain variable domain (VH) and/or a light chain variable domain (VL), both of which domains in turn comprise three Complementary Determining Regions (CDRs), wherein:
- VH CDR1, CDR2 and CDR3 comprise or consist of the sequences SEQ ID NO: 1, SEQ ID NO:2 and SEQ ID NO:3, respectively;
   and
- VL CDR1, CDR2 and CDR3 comprise or consist of the sequences SEQ ID NO:4, SEQ ID NO:5 and SEQ ID NO:6, respectively.

In a preferred embodiment, the antibody or fragment thereof comprises a heavy chain variable domain (VH) and a light chain variable domain (VL), wherein said heavy chain variable domain comprises or consists of the sequence SEQ ID NO:7, and/or said light chain variable domain comprises or consists of the sequence SEQ ID NO:8.

In a further preferred embodiment, the antibody fragment is scFv.

In an embodiment, the antibody is an immunoglobulin of IgG1, IgG3 or IgG4 isotype. The IgG1 isotype is preferred. As well as comprising the variable regions of the light and heavy chains, the antibody according to the invention can also comprise a constant region of the light chain containing a CL domain, and a constant region of the heavy chain containing the domains CH1-CH3 and a "hinge" region, optionally modified.

Another aspect of the invention regards a nucleic acid molecule encoding an anti-FV or FVa antibody or a fragment thereof as herein disclosed. Preferably, said nucleic acid molecule consists of a sequence which is selected from:
(i) the sequence SEQ ID NO.:9, encoding VH CDR1;
(ii) the sequence SEQ ID NO: 10, encoding VH CDR2;
(iii) the sequence SEQ ID NO: 11, encoding VH CDR3
(iv) the sequence SEQ ID NO: 12, encoding VL CDR1;
(v) the sequence SEQ ID NO: 13, encoding VL CDR2;
(vi) the sequence SEQ ID NO: 14, encoding VL CDR3;
(vii) the sequence SEQ ID NO: 15, encoding the VH chain; and
(viii) the sequence SEQ ID NO: 16, encoding the VL chain.

Another aspect of the invetion regards an expression vector comprising a nucleic acid molecule as herein defined, or a host cell comprising said vector.

The novel anti-factor VIVa antibody is useful not only as a therapeutic agent and particularly as a specific anti-coagulant, but it also provides a tool for diagnostic and research applications.

Hence, another aspect of the invention regards a diagnostic or therapeutic composition comprising an antibody or fragment thereof as herein provided.

The composition comprises pharmaceutically acceptable carriers and excipients, such as diluents, adjuvants, buffering agents, emulsifiers, humectants, solubilisation agents or other substances which allow or facilitate the administration of the antibody and its distribution in the body and delivery to the site of action, or which reduce its toxicity, increase its bioavailability or promote compliance by the individual to whom it is administered. For the selection of an excipient suitable for the applications specified herein, see the manual "Handbook of Pharmaceutical Excipients", 5th Edition, R. C. Rowe; P. J. Seskey and S. C. Owen, Pharmaceutical Press, London, Chicago. The composition can take the form of a solution, suspension, emulsion, tablet, capsule, microcapsule, liposome, powder, sustained-release formulation or lyophilizate. Suitable methods of administration include oral, sublingual, nasal and parenteral administration, and in particular intravenous, subcutaneous, intramuscular and intradermal administration.

In a preferred embodiment, the antibody or fragments thereof are used as anticoagulant agents inhibiting Factor V and/or Factor Va, particularly useful to reduce clot formation and associated thrombotic events in a subject in need thereof.

In a further embodiment, the antibody or fragments thereof are used *in vitro* to measure the levels of Factor V in a blood sample, for example using immunohistochemical, immunoenzymatic or immunoradiometric methods, such as radioimmunoassays, immunofluorescence assays or immunoenzymatic assays, or to isolate Factor V from a blood sample.

### Description of the Figures

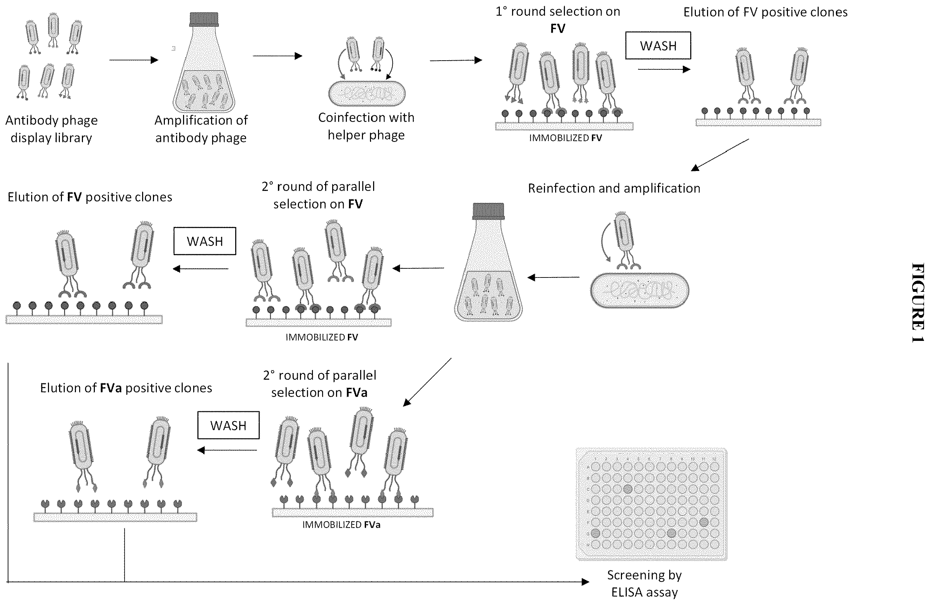

**Figure 1****.** Schematical representation of phage display selection strategy used for identification of anti-FV/FVa scFvs.

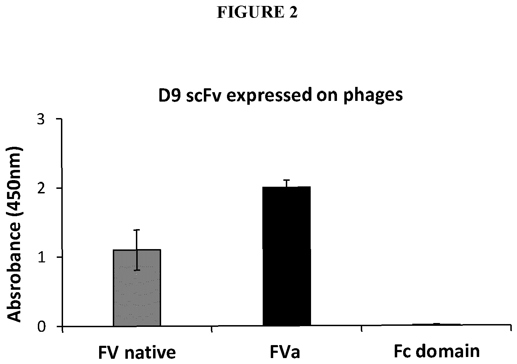

**Figure 2****.** ELISA assays of D9 phage on FV and FVa. Binding assay of D9 positive phage-scFv clone by ELISA on immobilized human active FVa or FV, coated on the plates at a concentration of 5 *mg*/mL. In parallel assays, Fc domain was used as negative control.

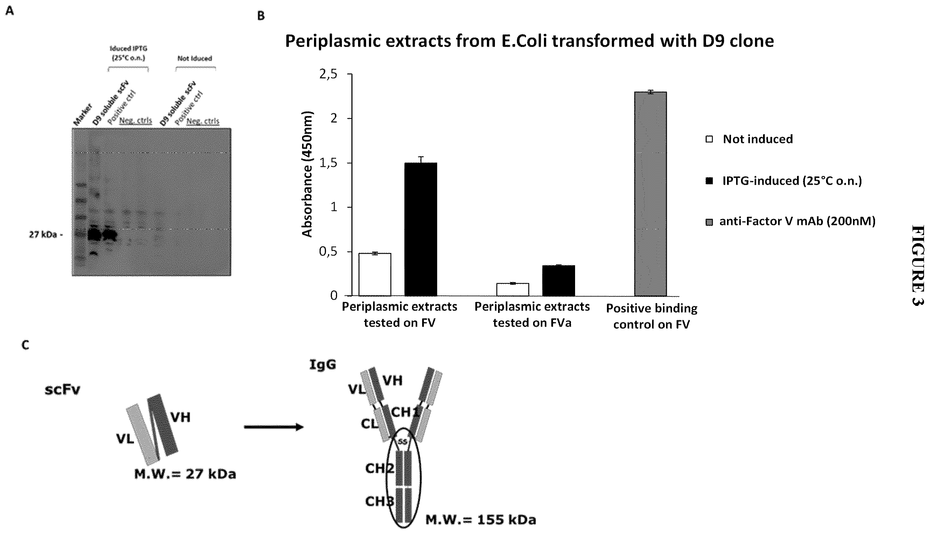

**Figure 3****.** Analysis of D9 as soluble scFv and conversion into full size mAb. **A.** Western blotting analysis of periplasmic exctract of the cells transformed with D9 clone, expressed in the absence (channel 1) or in the presence (channel 2) of IPTG, used for induction. The signal was detected by using the anti-c-myc antibody. **B.** In parallel, the periplasmic extracts, were tested on immobilized human FV or FVa by ELISA assays and the binding measured by using the anti-cmyc antibody. As positive control, a commercial anti-FV mAb was used. **C.** Schematic representation of the conversion of scFv into a fully human monoclonal antibody.

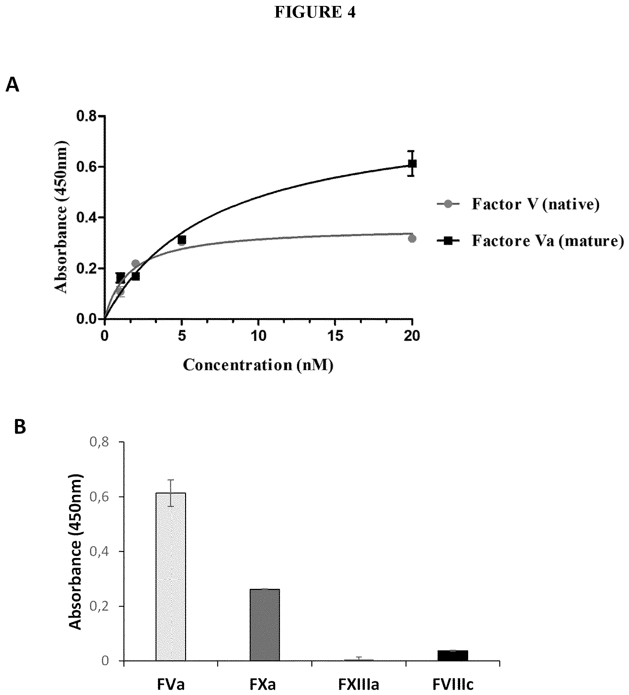

**Figure 4****.** Binding of D9 to FV and its cross-reactivity to other coagulation factors. **A.** Binding curves of D9 mAb (1-20 nM) to immobilized coagulation Factor V native precursor (grey circles) and Factor Va mature protein (black squares) by ELISA assays. **B** Evaluation of the cross-reactivity, by ELISA assays, of D9 mAb, at a fixed concentration of 20 nM, for different coagulation factors, coated on the plate at a fixed concentration of 5µg/ml. D9 was detected using an anti-Fab HRP-conjugated antibody. Binding values were reported as the mean of at least three determinations. Error bars depicted means ± SD.

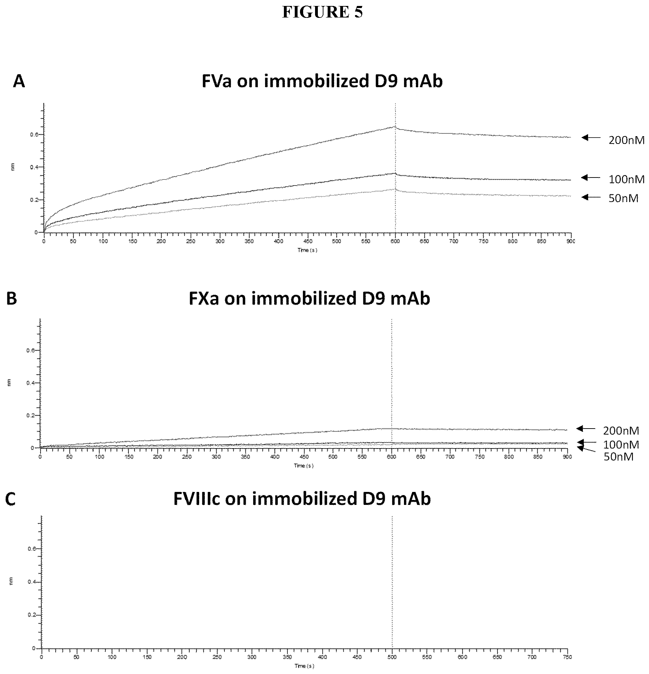

**Figure 5****.** Binding kinetics of D9 by BLI analyses. **(A)** Binding of the factor Va, tested as analyte, at increasing concentrations (50-200nM) on the immobilized D9 mAb, used as ligand, at the concentration of 2µg/ml. **(B)** Binding of the factor Xa or **(C)** factor VIIIc on the immobilized mAb, performed in parallel assays in the same conditions. The sensorgrams show the association and dissociation rates of the analytes.

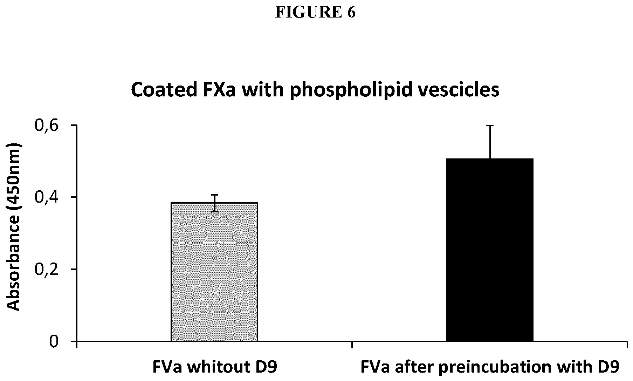

**Figure 6****.** Competitive ELISA assay to test the effects of D9 in the formation of the FVa-FXa complex. The coagulation factor Xa was coated on the plate at a concentration of ~2µg/ml, in the presence of phospholipid vescicles. Then, FVa at a concentration of 200nM alone (grey bar) or after preincubation with D9 (5:1 mAb molar excess) (black bar) was added. Binding values were reported as the mean of at least three determinations. Error bars depicted means ± SD.

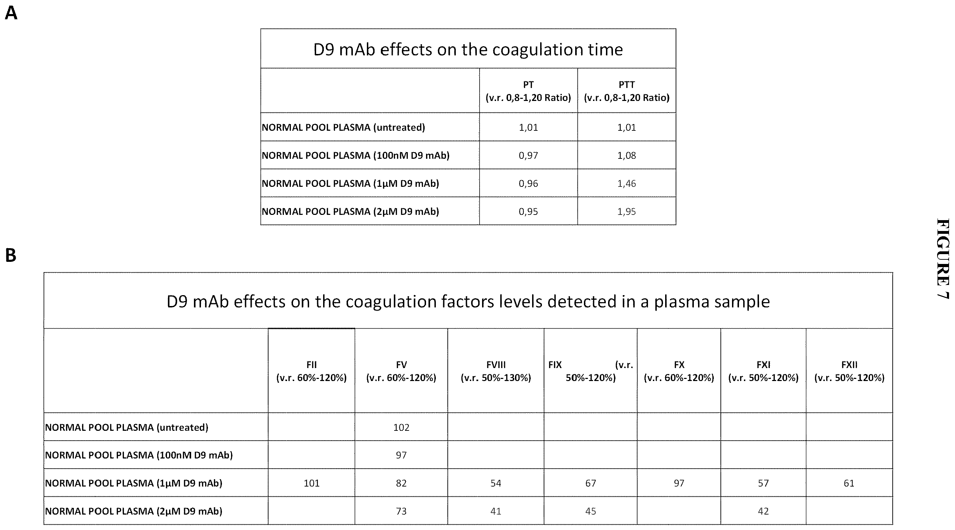

**Figure 7****.** Effects of D9 on blood coagulation. Functional assay on a plasma sample to measure the effects of the D9 mAb on the coagulation cascade. A plasma sample of 0.5mL was pre-incubated for 1h at room temperature with increasing doses of D9 (100 nM, 1 µM and 2 µM). **(A)** The prothrombin time and the partial thromboplastin time or **(B)** different coagulation factors levels were measured. An untreated sample was used as negative control.

### Detailed description of the invention

In order to isolate a scFv specific for FVa we designed a selection strategy, based on phage display technology, starting from a large human scFv phage library. In particular, to select a scFv able to specifically recognize both the precursor FV and the activated co-factor FVa, we planned parallel panning rounds on both human purified target proteins, either in native or activated form, derived from human blood plasma. In particular, the first panning round of the phage library was performed on immobilized native human Factor V, then the phages, eluted by the classical acidic elution method by lowering pH and used to infect E. Coli TG1 cells for amplification. The amplified pool of eluted phages was subjected to two consecutive parallel panning rounds performed on human FV or human FVa **(****Figure 1****),** to increase the possibility of obtaining a common sequence capable of recognizing both the protein forms. The screening of positive binders was performed by massive ELISA assays on the two immobilized proteins. We identified a strong positive clone, named D9, isolated by the combination of selection rounds on native and mature form, endowed with high specificity both for the FV and the active FVa factors. The D9 clone displayed on phages was identified from the 3^{rd} round on FVa and tested in triplicates on immobilized native FV or active FVa protein by ELISA assays, as well as on an unrelated protein such as the human Fc domain, used as a negative control, to verify the specificity of the binders for FV As shown in **Figure 2****,** no significant binding to the Fc was observed, whereas a strong binding signal was detected on FVa factor, thus confirming the efficiency of the selection strategy.

The DNA from the phage clone encoding the selected scFv was extracted by digestion with suitable restriction enzymes to confirm the presence of full size insert encoding scFv and then fully sequenced, as described in Materials and Methods.

We investigated whether the novel identified scFv could be expressed as a soluble protein and could retain the binding properties of the scFv displayed on phages. To this aim, the cDNA encoding the scFv was used to transform the bacterial strain SF110 and the expression was induced with Isopropyl β-d-1-thiogalactopyranoside (IPTG) over night (o.n.) at 25°C. The periplasmic extracts of the selected clone, before and after the induction with IPTG, were firstly analyzed by Western Blotting and then tested by ELISA assays by using an anti-c-myc antibody. As shown in **Figure 3A****,** the novel scFv was expressed as soluble protein of the expected molecular weight of 27 kDa. The periplasmic extract, after IPTG induction, was then tested to verify its binding specificity on human immobilized FV or FVa purified protein **(****Figure 3B****)** and fully confirmed its ability to bind to both the proteins also when the scFv was expressed as soluble protein. On the other hand, no binding was observed with the periplasmic extract not induced with IPTG, thus confirming that the binding to FV is mediated only by the expressed exogenous sequence cloned downstream to the IPTG inducible promoter. To obtain a bivalent full size mAb, the isolated scFv was converted into more stable antibody format (IgG1) by subcloning the variable domains in frame to human IgG1 Fc into mammalian expression vectors **(****Figure 3C**). The recombinant antibody was produced and purified from the conditioned medium of CHO transfected cells by Protein G affinity chromatography, as described in Materials and Methods.

Once purified, the mAb was firstly tested by ELISA assays at increasing concentrations on both the mature FVa and its native precursor FV, in order to confirm its specific binding to the target even after the conversion into full size mAb. The results, shown in **Figure 4A****,** indicate that D9 monoclonal antibody was able to efficiently recognize the mature FVa, and its native precursor FV, with an apparent affinity (K_{d}) of 3-4nM. To test the the cross-reactivity of D9 mAb for other coagulation factors, FVa, FVIIIc, FXa and FXIIIa were immobilized on the plate, and D9 was tested by parallel ELISA assays at the concentration of 20 nM, which was the saturating concentration for the binding to FVa. D9 showed a slight binding to the FXa, whereas no binding at all was observed to the FVIIIc and the FXIIIa **(****Figure 4B**).

To further characterize the binding properties of the novel D9 monoclonal antibody, we performed BioLayer Interferometry (BLI) analyses to accurately evaluate the binding kinetics of D9 to the FVa and FXa coagulation factors. Despite the absence of binding observed in the ELISA assays, we also decided to test again the binding of D9 to the FVIIIc, due to the significative sequence homology between FVa and FVIIIc, in order to definitively exclude a potential cross-reactivity by performing a different type of assay. To this aim, D9 was immobilized on the Protein A (ProA) biosensors at a concentration of 2µg/ml, then the binding of increasing concentrations of FVa (50-200 nM) was measured. The same experiment was performed in parallel by using FXa and FVIIIc in the same experimental conditions. As shown in **Figure 5A****,** D9 showed a significant binding to the FVa, with a K_{D} of 1.162⁻⁰⁸ (≃12nM); whereas a poor binding was detected to the FXa protein even when the coagulation factor was tested at the highest concentration of 200 nM **(****Figure 5B****).** No binding at all was detected for FVIIIc **(****Figure 5C****).** The different K_{D} value observed by BLI with respect to that measured by ELISA is due to the single-site (monovalent) binding affinity measured by BLI with respect to the bivalent one analyzed by ELISA, that is affected by avidity.

In order to investigate the mechanism of action of this newly-generated mAb, we performed competitive ELISA assays to test the effects of D9 on the binding between FVa and FXa, in the presence of phospholipids added in order to increase the interaction between the two factors, as previously reported in literature (*refs 1 Lindhout et al, Factor Va-Factor Xa Interaction. Effects of Phospholipid Vesicles of Varying Composition 2) Rosing et al, The role of phospholipids and factor Va in the prothrombinase complex*)*.* Firstly, the FXa mixed with the phospholipids vescicles was coated on the plate; then, the FVa, alone or preincubated with 5:1 molar excess of D9 for 1h and 30m at room temperature, was added. The results, shown in **Figure 6****,** indicate that the presence of D9 does not interfere in the binding between FVa and FXa.

After testing the binding properties of the novel D9 mAb, we wanted to evaluate its ability to affect the coagulation cascade in a functional assay. To this aim, the mAb was preincubated with a plasma sample at the indicated doses for 1 h at room temperature, and then the prothrombin time (PT) and partial thromboplastin time (PTT) were measured. The results, shown in **Figure 7A****,** indicate that D9 prolonged significantly the aPTT, whereas no significant effects were observed on PT; thus, we further investigated its effects on the intrinsic pathway by measuring the levels of the other factors involved. The obtained data, represented in **Figure 7B****,** show that D9 reduced the levels of FV in a dose-dependent fashion. The addition of D9 to the plasma was also able to reduce even more significantly the levels of several other coagulation factors: FVIII, FIX and FXI. These results suggest that the novel D9 mAb is able to interfere with the intrinsic pathway of the coagulation cascade.

### Materials and Methods

### Antibodies and human recombinant proteins

The following human recombinant proteins were used:
Native Human Coagulation Factor V (F5-5300H, Creative Biomart, Shirley, NY, USA); Human Factor Va Native Protein **(RP-43128),** Human Factor Xa Native Protein **(RP-43114),** Human Factor XIIIa Native Protein (RP-43123) all by Invitrogen, Rockford, IL, USA.

The human commercial antibodies used are reported below:
Monoclonal Antibody specific for Factor V (MA1-43005, Invitrogen, Rockford, IL, USA); HRP- conjugated anti-M13 Monoclonal antibody (27-9421-01, Cytiva, Wilmington, DE, USA); HRP- conjugated anti-cmyc antibody (130-092-113, Miltenyi Biotec, Bergisch Gladbach, Germany); anti-mouse IgG, HRP-linked antibody (7076, Cell Signaling, Danvers, MA, USA); anti-human IgG (Fab')2 HRP-conjugated goat monoclonal antibody (ab87422, Abcam, Biomedical Campus, Cambridge, UK).

### Bacterial strains, culture media and antibiotics

*E.Coli* TG1 bacterial strain was used for the production of phages; *E.Coli* SF110 bacterial strain was used for the expression of the scFvs as soluble proteins. Bacterial growth was carried out in the culture YT-Medium media (A0981, PanReac by AppliChem Darmstadt, Germany). The media were supplemented with 1% D-(+)-Glucose (G7528, Sigma-Aldrich T0440, St Louise MO). The antibiotics used were Ampicillin (A7492) at the concentration of 100 µg/ml and Kanamycin (A1493) at 25 µg/ml (all from AppliChem, Darmstadt Germany).

### Selection of scFv-phage clones.

Phagemid particles were isolated (043443.A3 Thermo Fisher Scientific, Waltham, MA, USA) (10¹³ cfu) from the TG1 trasformed with the library by using the M13-K07 helper phage (18311-019, Invitrogen, Rockford, IL, USA), as previously described (Sasso et al. mAbs 2018*,* Passariello et al. Sci rep 2021*,* De Lorenzo C et al. Clinical Cancer Res. 2002*).* Phage particles were purified and concentrated by two steps of precipitation with ice cold PEG 20% (043443.A3 Thermo Fisher Scientific, Waltham, MA, USA) containing NaCl 2,5 M and washed with 20 ml of sterile water. After an additional PEG/NaCl precipitation step, phages were resuspended in Phosphate-Buffered Saline (PBS), centrifuged at 12,000 rpm for 15 min at 4 °C, and stored at 4°C until use.

For each round of selection, phages were firstly blocked with a solution of PBS/Milk 5%. The selection was carried out, by incubating the phages for 2 h at 4°C by gently rotation with the protein Human Factor V coated 20 µg/ml on Nunc polypropylene tubes in a first round. After extensive washes with PBS, the bound phages were eluted with 76 mM citric acid (pH 2.5) in PBS for 5 minutes, and then neutralized with 1M Tris-HCl (pH 8.0). The recovered phages were amplified by infecting *E. coli* TG1 cell. The phagemid particles obtained from amplification were used for two following rounds of selection, performed in parallel either on Human Factor V or Human Factor Va immobilized at a concentration of 20 µg/ml on polypropylene tubes. Phages were then collected and stored at 4°C until use.

### Preparation of scFv-phages for ELISA and Analysis of positive clones

A TG1 culture was infected with the eluted phages (after 3 rounds of panning) and plated on 2xTY/agar containing 1% glucose and ampicillin (100 µg/ml). Individual clones were picked, transferred into 96-well plates and grown in 100 µl of 2xTY medium containing 1% glucose and ampicillin (100 µg/ml) for 18 hours by shaking at 37°C. The scFv-phages were produced by superinfection with M13-K07 helper phage for 1 hour at 37°C. The plates were then centrifuged at 1200 rpm for 30 minutes at 4°C to pellet the bacteria, and aliquots of 50 µl of scFv-phage-containing supernatants were used for ELISA.

Positive clones selected by ELISA were inoculated in 10 ml of 2xTY containing 100 µg/ml of Ampicillin and 1% glucose and grown up over night by shaking at 37°C to prepare the plasmids by using Wizard^{®} Plus SV Minipreps DNA Purification System (A1330, Promega, Madison, WI, USA). To verify the presence of cDNA encoding the single chain variable fragments, positive clones were digested with *Ncol* and *NotI* enzymes and analyzed by electrophoresis on 1% agarose gel. The nucleotide sequences encoding scFvs were determined by using suitable PCR primers by an internal facility (CEINGE, Napoli, Italy). The sequences were analyzed with a software for DNA and amino acid editing and alignments (*GENtle-software,* Magnus Manske, University of Cologne, Germany).

### Soluble scFv Expression and partial Purification

The plasmid containing the cDNA encoding the anti-FVa scFv was used to transform the competent bacterial strain of SF110 wich was, then, plated overnight at 37°C on a 2x YT Agar Petri dish supplemented with 100 µg/ml Ampicillin and 1% Glucose. Then, the single CFU was picked and inoculated at 37°C in 2x YT medium containing Ampicillin and Glucose at the concentration indicated above, until the OD600 reached 0.8. Cells were centrifuged at 3500 rpm for 15 min and resuspended in glucose-free 2x YT medium. The expression of soluble scFv was induced by adding isopropyl-1-thio-β-D-galactopyranoside (IPTG) (A4773, PanReac by AppliChem Darmstadt, Germany) at a concentration of 1 mM in the *E.coli* SF110 culture, which was then grown at 25°C overnight.

Cell cultures were centrifuged at 3500 rpm for 15 min, and a periplasmic extract was obtained by resuspending cells in a solution of B-PER Bacterial Protein Extraction Reagent (78248, Thermo Fisher Scientific, Waltham, MA, USA) with Protease Inhibitors 1X (11873580001, Roche Diagnostics GmbH, Mannheim, Germany), according to the manufacturer's recommendations.

### ELISA assays of D9 soluble scFv

To confirm the binding ability of the novel anti-FVa D9 scFv, ELISA assays were performed on Human Coagulation Factor V or Human Factor Va . The NuncTM flat bottom 96-well plate was coated with 5 µg/mL of Factor V or Factor Va Proteins (both from Invitrogen, Rockford, IL, USA) in a solution of 0.05 M NaHCO3 for 48 h at 4 °C. After blocking the coated 96-well plates with a solution of PBS/ Milk 5% for 1 h at 37 °C, the D9 soluble scFv was diluted in PBSBSA 3% and incubated for 2 h at room temperature by gently shaking. As a positive control, a commercial anti-Factor V mAb (MA1-43005 Invitrogen, Rockford, IL, USA) was used at a concentration of 200 nM. After the first incubation, extensive washes were carried out with PBS, then the plate was incubated for 1 h at RT with HRP-conjugated anti-cmyc or anti-mouse HRP-conjugated antibody, for the detection of soluble D9 scFv or commercial anti-FV mAb, respectively. After washes, the plates were incubated with 3,3',5,5'-tetramethylbenzidine (TMB Sigma-Aldrich T0440, St Louise MO). Absorbance at 450 nm was measured by the Envision plate reader (Perkin Elmer, 2102).

### Conversion of scFv into full size mAb

A synthetic gene containing the sequence encoding for the variable region of the light chain of the scFv and the constant region of the human lambda chain was engrafted in NotI and XhoI sites of the pCDNA 3.4 plasmid (Thermo Fischer Scientific) to generate the pCDN3.4 Light Chain expression vector. Similarly, a synthetic gene containing the sequence encoding for the variable region of the heavy chain of the scFv and the constant regions of the human IgG1 heavy chain was cloned in in the NotI and XhoI sites of the pCDNA3.4 plasmid to generate the pCDNA 3.4 Heavy Chain expression vector.

CHO cells were maintained in Dulbecco modified Eagle medium supplemented with 10% fetal bovine serum (Sigma-Aldrich), antibiotics (100 µg/mL penicillin, 100 µg/mL streptomycin), and 1 mM L-glutamine (Invitrogen). Cells were transiently transfected by using Calcium-phosphate. Briefly, 2.5 micrograms of each plasmid were added to 500 microliter of solution A (250 mM calcium chloride). Then, 500 microliter of solution B (1.4 mM sodium salt of H₂PO4⁻, 140 mM sodium chloride, and 50 mM Hepes pH 7.05) was added and the solution was briefly mixed by using a vortex. After one minute, one ml of the solution was added to the cells and the flask was gently shaken to unsure an even distribution of the transfection cocktail. After an overnight incubation, cells were collected, resuspended in serum-free medium (Opti-MEM, Gibco) and cultivated for additional 48 hours. Conditioned medium was then collected, and the secreted antibody was purified by using a protein-G Sepharose FPLC column.

### D9 mAb ELISA assays

To check the ability of D9 mAb to recognize FV either in its native and mature (FVa) form, the converted mAb was used to perform binding curves at increasing concentrations. To this aim, human Factor V or Factor Va proteins were immobilized on 96-well plate at 5 µg/ml for 48 h at 4°C, then D9 mAb was incubated at increasing concentrations (1- 20 nM) for 2 h at RT. After adding the HRP-conjugated anti-Fab antibody, the signal was detected by measuring the absorbance at 450 nm and the binding curves were obtained by using Prism (GraphPad Prism 5) tool.

To verify the cross-reactivity of D9 mAb for other coagulation factors, parallel ELISA assays were performed on Human Factor Va, Human Factor Xa, Human Factor XIIIa proteins immobilized on 96-well plates at 5 µg/ml for 48 h at 4 °C. After blocking with PBS/ Milk 5% for 1 h at 37 °C, the novel D9 mAb was incubated at a concentration of 20 nM for 2 h at RT. After extensive washes with PBS, the plate was incubated for 1 h at RT with HRP-conjugated anti-Fab antibody. Absorbance at 450 nm was measured by the Envision plate reader (Perkin Elmer, 2102).

### Competitive ELISA assays

To test the ability of D9 mAb to interfere with the formation of the complex between FVa and FX , competitive ELISA assays were performed in the presence of phospholipid vescicles, thatwere formed by sonicating a mixture of phospholipids with a mixture of 60% 1,2-dioleoyl-sn-glycero-3-phosphatidylcholine (DOPC, 15098) and 40% 1,2-Dioctadecenoyl-sn-glycero-3-Phosphatidylserine (DOPS, 29983) (both by Cayman Chemical, Michigan, USA) , as previously described (Lindhout et al, Factor Va-Factor Xa Interaction. Effects of Phospholipid Vesicles of Varying Composition)*.* The vescicles were then incubated with the FXa at the concentration of 200 nM for 1h at 37°C; after that, the mixture was diluted in NaHCO3 and it was used to coat a 96-well plate for 48h at 4°C.

Then, the binding of FVa was tested before or after a preincubation with the novel D9 mAb at a 5:1 molar excess (mAb:coagulation factor), for 1h and 30m at room temperature. The binding of FVa was detected by incubating the wells with mouse anti-Human FV monoclonal antibody for 2h at room temperature, followed by an anti-mouse IgG HRP-conjugated secondary antibody for 1h at room temperature. Finally, TMB was added to the wells, and HCl 1N was used to stop the reaction. The absorbance was measured at 450 nM.

### Biolayer interferometry (BLI) analyses

To investigate the binding kinetics of the novel D9 mAb either for Human Factor Va or Factor Xa, BLI analyses were performed by using the Octet R4 Protein Analysis System (Sartorius, Fremont, CA, USA). Biosensors carrying the protein A (18-5010, Octet ProA Biosensors, Sartorius, Fremont, CA, USA) were used to perform the assays, by following the manufacturer's recommendation as previously reported [Passariello et al. Int. J. Mol. Sci. 2023, 24, 10053].

Briefly, the ProA biosensors' tips were hydrated for 15 min in 200 µL of Kinetic Buffer (KB) 10X (0.1% BSA, 0.02% Tween, in PBS 1X). Then, D9 mAb was loaded at a concentration of 2 µg/mL, for a time interval of up to 200 s. After washing, the association step was carried out by dipping the biosensors for 600 s in a solution containing Human FVa or FXa, used as analytes, at increasing concentrations (50, 100, 200 nM). Then, the dissociation step was performed in KB buffer 10X for 300s, and finally the biosensors were regenerated according to the manufacturer's recommendations. The data were acquired and processed into the Octet Analysis Studio Software 13.0 [Manna et al. Cancers 2023 Nov 9;15(22):5345]*.*

### Effects of D9 on coagulation pathway in blood human samples

D9 mAb was preincubated with plasma samples (0.5 mL) at increasing concentrations (100 nM - 2 µm) for 1 h at room temperature. Routine clotting time tests, such as activated partial thromboplastin time

(aPTT) and prothrombin time (PT), and second level haemostasis assays, encompassing factors II (FII), factor V (FV), factor VIII (FVIII), factor IX (FIX), factor X (FX), factor XI (FXI) and factor XII (FXII) were then measured by the automated ACL Top 550 coagulometer (Instrumentation Laboratory Company, Bedford, MA, USA). Each factor activity is quantified by performing a modified PTT test: the plasma analyzed is diluted and added to a deficient plasma for the factor that would be studied. Correction of the clotting time of the deficient plasma is proportional to the correction (% activity) of that factor on the plasma analyzed, interpolated from a calibration curve.

## Claims

1. An antibody specifically binding to Human Factor V and Va, or a fragment thereof selected from Fab, Fab', Fab'-SH, Fv, scFv, (Fab')2, diabodies (dAb) or single domain antibody (sdAb), wherein said antibody or fragment thereof comprises a heavy chain variable domain (VH) and/or a light chain variable domain (VL), both of which domains in turn comprise three Complementary Determining Regions (CDRs), wherein:
- VH CDR1, CDR2 and CDR3 comprise or consist of the sequences SEQ ID NO: 1, SEQ ID NO:2 and SEQ ID NO:3, respectively;
and
- VL CDR1, CDR2 and CDR3 comprise or consist of the sequences SEQ ID NO:4, SEQ ID NO:5 and SEQ ID NO:6, respectively.

2. The antibody or fragment thereof according to claim 1, wherein:
- the heavy chain variable domain (VH) comprises or consists of the sequence SEQ ID NO:7;
and/or
- the light chain variable domain (VL) comprises or consists of the sequence SEQ ID NO:8.

3. An antibody fragment according to claims 1-2, which is scFv.

4. An antibody according to claims 1-2, which is an immunoglobulin G of IgG1, IgG3 or IgG4 isotype.

5. A nucleic acid molecule encoding an antibody or fragment thereof according to claims 1-4.

6. A nucleic acid molecule according to claim 5, wherein, independently from each other:
(i) the sequence encoding VH CDR1 is SEQ ID NO:9;
(ii) the sequence encoding VH CDR2 is SEQ ID NO: 10;
(iii) the sequence encoding VHCDR3 is SEQ ID NO: 11
(iv) the sequence encoding VL CDR1 is SEQ ID NO: 12;
(v) the sequence encoding VL CDR2 is SEQ ID NO: 13;
(vi) the sequence encoding VL CDR3 is SEQ ID NO: 14;
(vii) the sequence encoding the VH chain is SEQ ID NO: 15;
(viii) the sequence encoding the VL chain is SEQ ID NO: 16.

7. An expression vector comprising a nucleic acid molecule according to claims 5-6, or a host cell comprising said vector.

8. A diagnostic or therapeutic composition comprising an antibody or fragment thereof according to claims 1-4.

9. An antibody or fragment thereof according to claims 1-4, for use in the inhibition of Factor V and/or Factor Va in a subject in need thereof.

10. An antibody or fragment thereof for use according to claim 9, wherein said inhibition of Factor V and/or Factor Va reduces clot formation and associated thrombotic events.

11. An anticoagulant agent comprising an antibody or fragment thereof according to claims 1-4.

12. The use *in vitro* of an antibody or fragment thereof to measure the levels of Factor V in a blood sample or to isolate Factor V from a blood sample.
